# EUROPEAN PATENT SPECIFICATION

(11) **EP 2 477 637 B1**
(45) Date of publication and mention of the grant of the patent: **20.04.2016**
(21) Application number: 10754299.5
(22) Date of filing: 03.09.2010
(51) Int. Cl.: A61K 35/74, A23L 33/135, A61P 17/00, A61P 17/10, A61P 17/08, A61P 17/04, A61P 17/16

(54) **PROBIOTIC LACTOBACILLUS RHAMNOSUS STRAIN AND ORAL AND TOPICAL USES THEREOF**
EIN PROBIOTISCHER LACTOBACILLUS RHAMNOSUS STAMM UND ORALEN UND TOPISCHEN VERWENDUNGEN DAVON
UNE SOUCHE DU PROBIOTIQUE LACTOBACILLUS RHAMNOSUS ET UTILISATIONS ORALES ET TOPIQUES DE CELLE-CI

(30) Priority: 08.09.2009 IT MI20091547
(43) Date of publication of application: 25.07.2012
(73) Proprietor: Giuliani S.p.A., 20129 Milano (IT)
(72) Inventor: GIULIANI, Giammaria, Milano (IT); BENEDUSI, Anna, I-20124 Milano (IT); MASCOLO, Antonio, I-20126 Milano (IT)
(74) Representative: Coppo, Alessandro
(86) International application number: PCT/EP2010/062993
(87) International publication number: WO 2011/029784

(56) References cited:
- WO-A1-2005/060937
- FR-A1- 2 508 282
- BOGUNIEWICZ M ET AL: "Current management of atopic dermatitis and interruption of the atopic march" JOURNAL OF ALLERGY AND CLINICAL IMMUNOLOGY 200312 US LNKD- DOI:10.1016/J.JACI.2003.09.031, vol. 112, no. SUPPL. 6, December 2003 (2003-12), pages S140-S150, XP002580332 ISSN: 0091-6749
- ORMEROD A D: "What is new in therapy?" BRITISH JOURNAL OF DERMATOLOGY 2001 GB LNKD- DOI:10.1046/J.1365-2133.2001.04503.X, vol. 145, no. 5, 2001, pages 691-695, XP002580333 ISSN: 0007-0963
- FÖLSTER-HOLST R ET AL: "Prospective, randomized controlled trial on Lactobacillus rhamnosus in infants with moderate to severe atopic dermatitis" BRITISH JOURNAL OF DERMATOLOGY, WILEY-BLACKWELL PUBLISHING LTD, GB LNKD- DOI:10.1111/J.1365-2133.2006.07558.X, vol. 155, no. 6, 1 December 2006 (2006-12-01), pages 1256-1261, XP002534276 ISSN: 0007-0963
- TANAKA A ET AL: "Oral supplementation with Lactobacillus rhamnosus CGMCC 1.3724 prevents development of atopic dermatitis in NC/NgaTnd mice possibly by modulating local production of IFN-[gamma]" EXPERIMENTAL DERMATOLOGY 2009 BLACKWELL PUBLISHING LTD GBR LNKD- DOI:10.1111/J.1600-0625.2009.00895.X, vol. 18, no. 12, 23 June 2009 (2009-06-23) , pages 1022-1027, XP002580334
- DATABASE WPI Week 200923 Thomson Scientific, London, GB; AN 2009-E60663 XP002580335 & KR 2008 010 095 A (KOREAN COSMETICS CO LTD) 14 November 2008 (2008-11-14)

## Description

### FIELD OF THE INVENTION

The present invention relates to a probiotic bacterial strain and the oral and topical uses thereof.

The present invention originates from and is applicable in the nutrition field as well as in the medical and cosmetic products fields.

In particular, the present invention relates to a selected strain of *Lactobacillus rhamnosus* and its uses in the food-nutrition, cosmetics and/or medical fields.

### TECHNICAL BACKGROUND OF THE INVENTION

As it is well known, the probiotic bacteria belong to bacteria kinds that can be safely long-time used in human beings and that can have beneficial effect on the host organism, if assumed in suitable quantities.

The use of probiotic bacteria in dietary supplements or dietary products is widely documented in the literature.

Typical examples of probiotic bacteria commonly used in nutritional and dietary fields belong to the Lactobacillus kind.

Such Lactobacilli are commonly administered orally in a suspension or lyophilisate form in order to integrate the intestinal bacterial flora and to restore optimal conditions for the intestinal bacterial flora development.

The probiotic products market has grown considerably in the last years, due to the properties found in some kinds of microorganisms, and to their use safety in the dietary and food fields.

However, even today there is a constant and growing need for new products or supplements based on probiotic microorganisms, whose use has no contraindications and which are indicated for use in specific fields, like the dermatologic and cosmetic ones.

Consequently, it is one of the general objects of the present invention to provide a selected probiotic microorganism having properties which are useful for dermatological or cosmetic applications, and which is suitable for either oral or topical administration.

Another object of the invention is to provide nutritional/dietary preparations for oral administration or compositions for topical use containing a selected strain of *Lactobacillus,* which exerts an anti-inflammatory action on the skin.

A further object of the present invention is to provide food supplements for oral administration or cosmetic compositions for the topical use containing a selected strain of Lactobacillus, suitable for preventing or treating the most common inflammatory or allergic skin affections.

### SUMMARY OF THE INVENTION

With these objects in view, the Applicant has found and selected a strain of *Lactobacillus* belonging to the *rhamnosus* species that, unexpectedly, exerts a strong anti-inflammatory activity when administered orally or topically.

According to a first aspect of the present invention, a specific probiotic bacterium is thus provided, belonging to the *Lactobacillus* strain, *rhamnosus* species, registered by the Applicant (Depositor) at a suitable Microorganism Deposit Center and given the accession number LMG P-25211 by the International Depositary Authority.

Specifically, this selected strain has been registered by the same Applicant for patenting procedure, under the Budapest Treaty, at the Belgian Coordinated Collections of Microorganisms - BCCM - LMC; at the University of Gent, K.L. Ledeganckstraat 35, B-9000 Gent, Belgium, on 11 February 2009, under the Accession number LMG P-25211.

The microorganism identification reference provided by the depositor (that is Giuliani Spa, Milan) at the register is *Lactobacillus rhamnosus* T12.

The Applicant has noted in this selected *Lactobacillus rhamnosus* strain an unexpected anti-inflammatory activity, that is not found in known probiotic microorganisms, the most common lactobacilli available included.

The Applicant has found that the *Lactobacillus rhamnosus* strain LMG P-25211 of the invention is surprisingly capable of enhancing considerably the expression of the anti-inflammatory protein PPARy and, on the other hand, of reducing the expression of the enzyme COX-2, a pro-inflammatory protein, in basal conditions.

This activity, at such high rate, is not evident for other known lactobacilli, even belonging to the same *rhamnosus* species.

Specifically, it has been found that the *Lactobacillus rhamnosus* strain LMG P-25211 of the invention has an unexpectedly strong anti-inflammatory activity, exerted by the induction of anti-inflammatory cytokines, in particular the IL-10 and IL-6 cytokines.

According to an aspect of the invention the *Lactobacillus rhamnosus* strain LMG P-25211 is thus provided for use in the treatment or in the prevention of any inflammatory and/or allergic affections, in particular at the skin.

According to an aspect of the invention, the strain of the invention founds application in the cases, where an inflammation process is under way and the inflammatory reaction is to be alleviated, in particular if at the level of the skin.

It has also been unexpectedly found that the topical application of *Lactobacillus rhamnosus* LMG P-25211 determines, on the keratinocytes, a significant reduction of the basal levels of the COX-2, a protein involved in the humoral inflammatory response. This action is specific for the selected microorganism of the invention, and is not evident for other strains of *Lactobacillus rhamnosus* of the prior art.

Typically, the strain of the invention can be conveyed to the action site of the human body, in order to obtain the anti-inflammatory effect, by means of a suitable physiologically acceptable carrier.

The term physiologically acceptable carrier means any substance that is aimed at supporting the strain of the invention and that does not produce harmful effects to the health, when administered to a human being or an animal.

In one embodiment, the physiologically acceptable carrier can be any food, pharmaceutical or cosmetic product, suitable for the oral or topical administration and to which the microorganism of the invention or its supernatant culture can be added.

By way of example, in the case of oral administration of the strain of the invention, one or more of the carriers commonly used in the formulation of the dietary supplements or the dietary products can be used, or simply one or more food articles suitable for carrying the microorganism such as, for example, milk and its derivatives, yogurt, cheese, fermented milk, cream, ice-cream, fermented cereals products, powder milk products, health food for children or animals, oral bacterial suspensions, dry or humid food supplements.

In another example, the strain of the invention can be added to cosmetic preparations or bases such as pastes, lotions, solutions, gel, fluids for the body, creams, specifically solar creams, after sun creams, anti age creams, ointments. The microorganism can be included in these preparation in living form, half active or in deactivated form (tyndallized), for example as lyophilized powder. In one embodiment, the cosmetic product can include also supernatant cultures of the strain of the invention, optionally in the concentrated form.

Typically, when the strain of the invention is administered to an individual, its anti-inflammatory action will depend on the dose. For example, 10⁵ to 10¹² microorganisms/g of product can be included, in which the microorganism can be vital or not.

According to an embodiment of the invention, a preparation or dietary supplement or dietary product is thus provided for oral taking, comprising an effective amount of *Lactobacillus rhamnosus* having the accession number LMG P-25211 in a physiologically acceptable carrier.

The administration of the preparation according to this embodiment is indicated for use in the prevention or treatment of any inflammatory or allergy state of the target organism (human or animal being), in particular when this form occurs at dermatological level.

According to an embodiment of the invention, a dietary supplement or dietary product is thus provided for oral administration, comprising an effective amount of *Lactobacillus rhamnosus* having accession number LMG P-25211 for the use in the treatment or prevention of an inflammatory and/or allergic skin infection.

In particular, the preparation according to this embodiment can be administered to treat or prevent a skin affection or disease, for example acne, atopic and/or seborrhoeic dermatitis, eczema and in general sensitive skin.

For example, the preparation containing the strain of the invention, when taken orally, reduces the inflammatory state which typically accompanies acne, thus determining considerable alleviation of its symptoms and additional itching.

The *Lactobacillus rhamnosus* strain LMG P-25211, when supported by a suitable carrier, can be used in the topical application in any situation, in which the local application of a substance having anti-inflammatory and/or anti-allergic effect, is required.

According to an embodiment of the invention a composition is thus provided for local use for the treatment and/or prevention of an inflammatory or allergic skin infection, comprising an effective amount of *Lactobacillus rhamnosus* LMG P-25211 in a physiologically acceptable carrier.

Within the scope of this embodiment, the composition for local use can be suitable formulated as a cosmetic or medicine.

According to an embodiment, the composition of the invention for topical application can take the form of a cream, emulsion, ointment, gel, lather, liniment, powder, solution or aqueous suspension, oily solution or suspension or biphasic solution or suspension.

By way of example, the composition for local use of the invention can be produced in solid form, for instance in the form of cream, such as face or body creams, or solar creams, sticks, transdermal patches, make-up products (foundation, powder, blusher, eye-shadow, mascara, eyeliner, lip liner), or in liquid form, for instance hydrophilic and/or hydroalcoholic lotions, milks, oleolites, shampoos, bath foams, sprays, dispersions, suspensions, or in semisolid form, for instance oil in water or water in oil emulsions, serums, hydrophilic or lipophilic gels, hydrophilic or oily make-up removers.

The composition of the invention for local use can include one or more physiologically or cosmetically acceptable excipients, typically used in the formulation of preparations for local application.

By way of example, the topical use formulation can include excipients, thickening agents, vitamins, aggregating agents, antimicrobial agents, surface-active agents, pads, humectants, preservatives, for example of the type indicated in the following examples.
In one embodiment, the topical use formulation of the invention incorporates also one or more substances having medical or pharmaceutical or cosmetic action, suitable for oral or topical administration.

### BRIEF DESCRIPTION OF THE DRAWINGS

The Figures provides further details of some aspects of the present invention. In the Figures:
Fig. 1 refers to the determination of pro-inflammatory systemic action IL-6, cytokine.
Fig. 2 refers instead to the release of the IL-10 anti-inflammatory cytokine by the keratinocytes.
Fig. 3 illustrates the Western Blot relating to determination of the PPARα expression.
Fig. 4 illustrates a Western Blot relating to determination of the PPARy expression.
Fig. 4a illustrates the quantification of the PPARy expression by the keratinocytes.
Fig. 5 illustrates a Western Blot relating to determination of the PPARδ expression.
Fig. 6 illustrates a Western Blot relating to determination of the COX-2 expression.
Fig. 7 illustrates the βactina expression.

### DETAILED DESCRIPTION OF THE INVENTION

Further additional characteristics of the invention are described in the following detailed description of the invention that includes the illustration of some tests carried out to select the specific strain of *Lactobacillus rhamnosus* and to check its properties in nutrition, cosmetic, dietary, medicinal fields.

### I. Activities and properties of Lactobacillus rhamnosus LMG P-25211

In particular, in order to check the anti-inflammatory activities of the strain of the invention, an *in vitro* model of human keratinocytes in culture was used. The tests were carried out using a living and vital strain (*Lactobacillus rhamnosus* LMG P-25211, later on identified also as T12) as a positive reference in order to verify the superimposability of data with those obtained previously *in vivo.*

### Preparation of the tyndallized bacterial strain

The tyndallized bacterial culture, to be used during some significant tests, was prepared beginning from a fresh culture of the *Lactobacillus rhamnosus* strain LMG P-25211, incubated in MRS for 16 hours at 37°C. Such a culture was subjected to a decimal count in order to determine the concentration of living cells. Such a culture was then centrifuged and the related pellet, re-suspended in an equal volume of growth medium for the keratinocytes, was heat treated at 70°C for 30 min, in accordance with the industrial tyndallization practice. Then, the culture was subjected, after the treatment, to a decimal count to estimate the possible residual cellular vitality. Table 1 reports the obtained count values.

**Table 1 - Decimal count (CFU/ml) of CBA-L93 in living and vital form and in tyndallized form**

| | Count (CFU/ml) |
|---|---|
| vital LMG P-25211 | 1,5 x 10⁹ |
| tyndallized LMG P-25211 | 8,9 x 10⁵ |

### Culturing and propagation of the keratinocytes.

The utilization model of a line of human keratinocytes in culture was deduced from the tests carried out previously on the vital strain. Indeed, the model was considered, also in this case, pertaining to the comparison of the immune-modulating effects obtained from the strain partially inactivated by heat and from the one in vital form.

### Carrying out of the tests by means of co-incubation of the keratinocytes with the tyndallized strain to be tested

The strain *L.rhamnosus* LMG P-25211 (T12), double-cultivated in selective ground, was taken in stationary growth phase and prepared for the tests by washing of the cellular pellet with PBS (phosphate buffered saline) in order to remove the exhausted culture ground. As it has already been noted, the choice to use living cells in stationary phase was based on observations of some authors, with regard to skin pathogens, according to which bacterial cells in exponential growth phase demonstrate smaller immune induction with respect to the stationary phase.

The washed cellular pellets were then re-suspended in an equal volume of the growth medium of the keratinocytes. One of the so obtained suspensions was heat treated in order to obtain the tyndallized (killed T12 - kT12).

Then both types of suspension (T12 and kT12) were put in contact with human keratinocytes in culture (108 cfu/ml, MOI 1:50). In parallel, the keratinocytes were pre-treated with lipopolysaccharide (LPS) 10 pg/ml for 30 minutes and then incubated with bacteria strains, as described above. After 1 hour incubation, the cells were washed and the growth medium restored.

Consequently, the cellular culture was prolonged for further 3 hours (for Western blot) or 24 hours (for ELISA assays).

When the incubation was completed, the cells were lysed in RIPA buffer and the cellular lysate was analyzed by means of Western Blotting, so as to determine PPARα, PPARδ, PPARy and COX2. Whereas, the growth medium was tested by ELISA, to estimate the levels of IL-6, IL-10 and IL-1δ cytokines.

### Preparation of the ELISA assays for determining the release of (1L-6, IL-10 and 1L-1β) cytokines

The growth medium of the keratinocytes was tested by ELISA, to estimate the levels of IL-6, IL-10 and IL-1δ cytokines.

Like in the assays carried out before, the IL-1δ cytokine has appeared to be non dosable in all the tested samples.

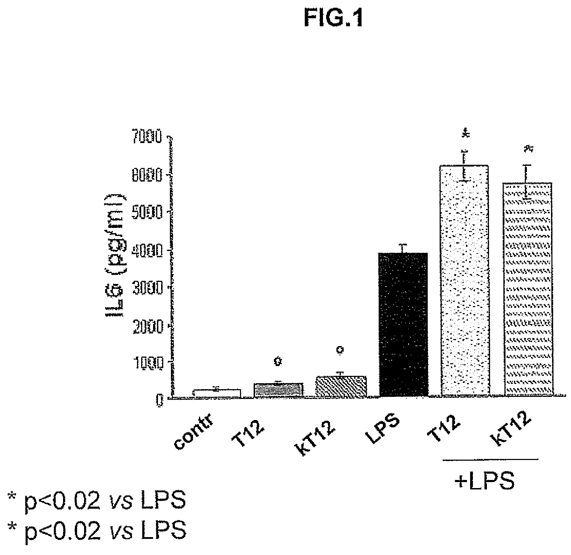

With regard to the IL-6, as illustrated in Fig. 1, in basal conditions, the strains LMG P-25211 (T12), *in vivo* or tyndallized, have made the keratinocytes increase the production and secretion of this cytokine with respect to the control, confirming the data of the previous tests. Due to the inflammatory conditions, as in the stimulation with LPS case, the production of IL-6 has considerably increased.

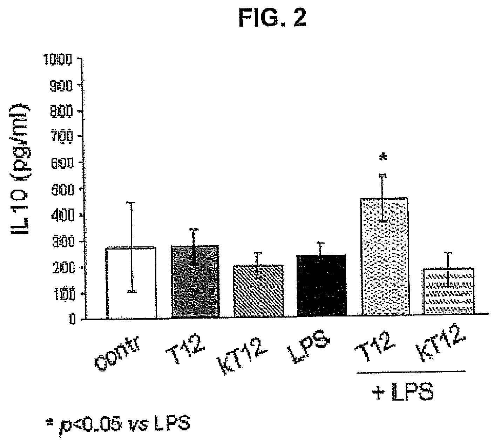

In basal conditions, the vital and tyndallized strains LMG P-25211 (T12) do not modify considerably the production of IL10 with respect to the control, whereas in inflammation conditions (due to the stimulation with LPS) the vital strain LMG P-25211 (T12) is capable of increasing the IL10 production and secretion, unlike the tyndallized strain. Fig. 2 specifically shows the delivery of the anti inflammatory cytokine IL-10 from keratinocytes.

### 1.4 In vitro evaluation of the induction of the PPAR-α. PPAR-δ e PPAR-γ and COX-2 mechanism by means of Western blotting

Twenty µg of the total proteins obtained after the lysis of the keratinocytes stimulated with LPS and/or with the bacterial strains were fractionated by means of SDS-PAGE (7,5% w/v).

Then, the proteins have been transferred onto the PVDF membrane.

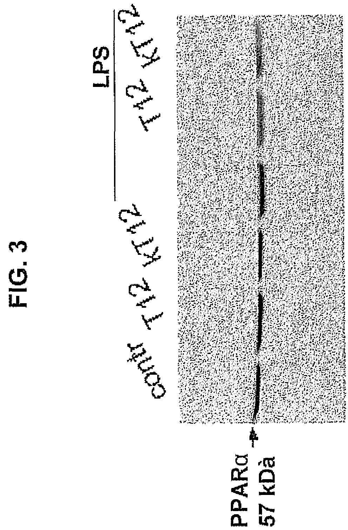

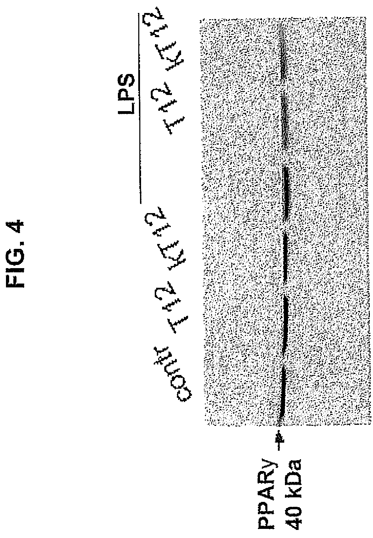

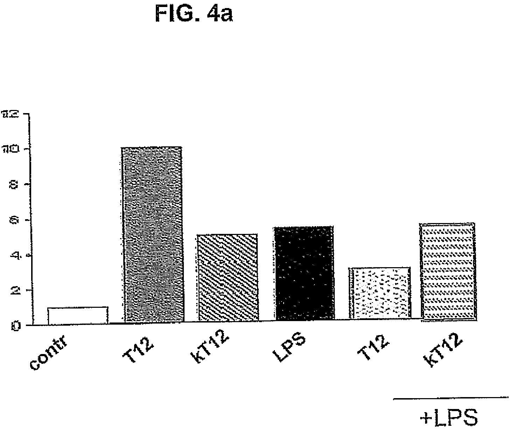

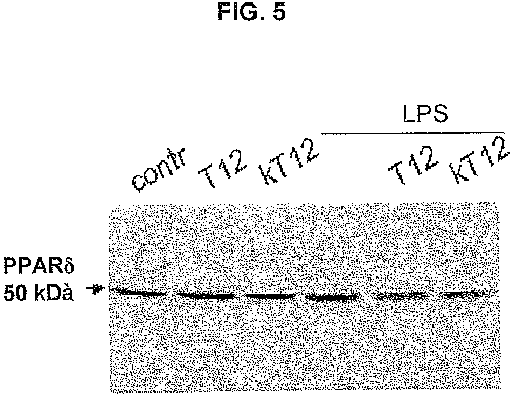

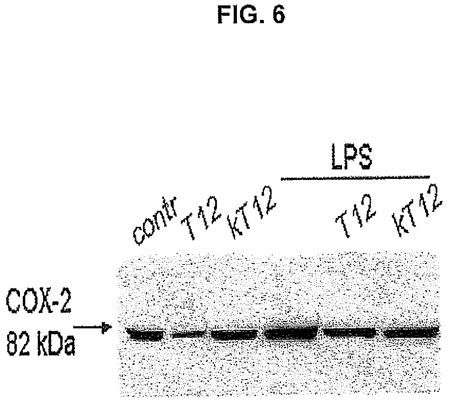

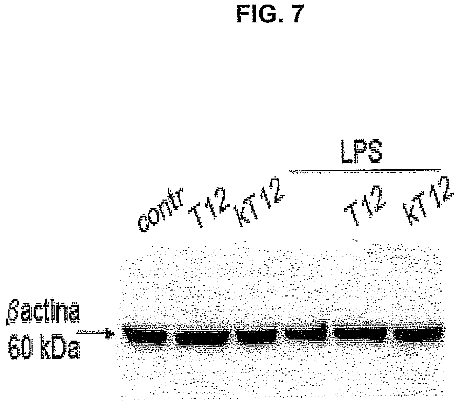

With reference to the previously mentioned Figures, in order to evaluate the expression of various proteins, such membrane was then incubated with antibodies specific for recognition of PPARα (Fig. 3), PPARy (Fig. 4), PPARδ (Fig. 5), COX-2 (Fig. 6). On the contrary, loading of the same quantities of protein for the different samples was confirmed by means of incubation of the membrane with a antibody directed against the human βactina, the protein expressed by the cells independently with respect to the added stimuli (Fig. 7).

Western Blot relating to the detection of the expression of PPARα is illustrated in Fig. 3.

The strain LMG P-25211 (T12) in living and vital form does not seem to modify the PPARα expression, as in Figure 3, in basal conditions after 4 hours incubation. The expression of such protein is not modified even by the strain LMG P-25211 (T12) subjected to tyndallization (kT12). Moreover, the PPARα levels are not varied after incubation with LPS (10 µg/ml) with or without treatment with the bacterial strains, confirming the observations of the previous experiments.

The data obtained from these experiments confirm the increase of the PPARy expression (Fig. 4 shows a Western Blot relating the determination of expression of PPARy), due to incubation of the keratinocytes with the strain LMG P-25211 (T12), as reported in the previous experiments. A densitometric analysis was carried out in order to quantify such expression variations. Specifically, Fig. 4a illustrates a quantification of the PPARy expression by the keratinocytes. The scale 0 to 12 of Fig. 4a represents an arbitrary unity of the relative expression.

On an arbitrary scale, the strain LMG P-25211 (T12) has increased the PPARy expression 10 times with respect to the control (not stimulated keratinocytes). Whereas the tyndallized strain LMG P-25211 (T12) has increased the PPARy expression 5 times with respect to the control. Both the vital strain and the tyndallized one do not affect significantly the PPARy expression in presence of LPS.

Confirming the previously obtained data, incubation with the vital strain LMG P-25211 (T12) has not changed in any way the PPARδ protein expression in the keratinocytes in absence or in presence of LPS, as it results from Fig. 5 (Western Blot of PPARδ). In the same way, the tyndallized strain LMG P-25211 (T12) has not evidently modified the expression of such protein.

The basal levels of COX-2, the protein involved in the synthesis of the pro-inflammatory prostaglandins, have been significantly reduced in the keratinocytes in basal conditions, following to incubation with the vital strain LMG P-25211 (T12). Western Blot is shown in Fig. 6. On the contrary, incubation with the tyndallized strain has not modified the expression of such enzyme with respect to the control cells.

As a result of the stimulation with LPS (inflammation condition), both the vital and the tyndallized strain LMG P-25211 (T12) have shown a similar effect in the reduction of the COX-2 levels to values comparable with those of the control cells.

The expression levels of βactina (Fig. 7) were assayed in order to point out equal loading for each of the tested samples for the method internal check.

The results obtained during the present analysis reveal a clear anti-inflammatory activity for the strain LMG P-25211 (T12) in living and vital form, as demonstrated by its capacity to increase the expression of anti-inflammatory PPARy protein and, on the contrary, to reduce the expression of the COX-2 enzyme, pro-inflammatory protein, in basal conditions. Moreover, this action is maintained and confirmed also after the stimulation with LPS. Further to tyndallization, the anti-inflammatory action of the strain LMG P-25211 (T12) results less evident, yet still important, in the reduction of the COX-2 levels, whereas the effects on the PPARy are not maintained.

The reported experiments have not revealed significant effects on the PPARα, after 4 hours incubation, either with the vital strain or with the tyndallized one.

As far as the cytokines are concerned, the effect on the IL-6 was confirmed, with superimposable effects between the vital and tyndallized strains. This datum is extremely interesting, since the IL6 is considered not only a pro-inflammatory cytokine, but is also regarded as a very important soluble factor for cellular regeneration and proliferation. On the other hand, as far as IL-10 is concerned, the vital strain has appeared to be more efficacious to increase the cytokine production in presence of LPS with respect to the tyndallized strain.

### II. Identification of Lactobacillus rhamnosus LMG P-25211

The bacterial strain of the invention belongs to a safe long-time use kind and is particularly suitable for the preparation of a form for topical application for treatment of local inflammatory skin phenomena in sensitive skin.

In order to select the strain, three different steps were carried out, aimed at checking the nature of the immune-stimulating effect present in a group of bacterial strains on human keratinocytes kept in culture and then passing to the selection of a single bacterial strain suitable for the application under examination, as well as evaluating the technological parameters capable of affecting the bacterial stability in the commercial formulation by testing its composition.

### Step 1: Bacterial strain isolation for subsequent characterization steps

The following bacterial strains, isolated from healthy child and adult feces, were identified from the taxonomic point of view by molecular methods and subjected to the subsequent analytical steps:
*Lactobacillus gasseri* T2
*Lactobacillus paracasei* 6/11
*Lactobacillus gallinarum* G3
*Lactobacillus rhamnosus* T12
*Lactobacillus crispatus* MU5

The selection of such bacterial isolates was caused by the need to test a wide range of different bacterial species within a bacterial group (generally *Lactobacillus*) with "a long story of safe use", which at the same time would assure good levels of biomass production.

### Step 2: Assessment of isolated strain capabilities in terms of pro- and anti-inflammatory immune activation, production of active molecules.

### 1.2.1 Immune activation

The realization of what is provided by the present activity, was obtained by selecting the human keratinocytes in culture as an ideal substrate for the preliminary determinations, necessary for evaluating the efficaciousness of a cosmetic preparation aimed at treating skin irritations by means of living lactic bacteria. In fact, being disseminated in the external skin layer (epidermidis), the keratinocytes represent the first skin defense line with respect to the external environment and can induce the cytokines and chemokines secretion to carry the alarm message to the skin deeper layers, generating the inflammatory response. During their evolution, they migrate from the deeper layers to those more superficial, with a progressive deposition of keratin, responsible for the protective action.

The human primary keratinocytes can be cultivated *ex-vivo* in laboratory and destined for the co-colture tests with bacterial strains in order to identify the nature of the immune response induced by the latter. The literature reports information about such tests with skin pathogens (e.g. *Propionibacterium acnes*) in order to evaluate the nature of the immune response generated by the pathogen on human skin. On the contrary, there is little information about the action related to the potential skin "probiotic" agents, since such an application is new.

The bacterial strains listed in the paragraph 1.1 were cultivated in selective ground, taken in stationary growth phase and prepared for the tests by washing of the cellular pellet with PBS (phosphate buffered saline) in order to remove the exhausted culture ground. The choice to use living cells in stationary phase was caused by observations of some authors, with regard to skin pathogens, according to which bacterial cells in exponential growth phase demonstrate smaller immune induction with respect to the stationary phase.

Then, the washed culture was re-suspended with PBS and put in contact with a monolayer of human keratinocytes in culture in active proliferation phase for 3 and 24 hours. The quantity of the used bacterial culture was in the order of 108 CFU per ml of cellular suspension.

When the contact period has finished, the supernatant of keratinocytes was subjected to the determination of the contents in interleukins 10, 6 and 1 (IL-10, IL-6, IL-1) by means of special Elisa kits (enzyme-linked immunosorbent assay). The obtained results are summarized in Tab. 1

| | Table 1 - Production of Interleukins 10, 6 and 1 by bacterial strains under examination | | | | |
|---|---|---|---|---|---|
| | **Strain** | **IL-10 (pg/ml)** | **IL-6 (pg/ml)** | **IL-1** | **Proliferation** |
| | | 24 h | 24 h | 24 h | 24 h |
| | Control | 437.7 | 478.5 | Non dosable | 100% |
| | MU5 | 627.9* | 604.3* | Non dosable | 123% |
| | G3 | 585.1 | 640.7* | Non dosable | 117% |
| | T2 | 578.7 | 576.7 | Non dosable | 135% |
| | T12 | 597.9 | 664.3 * | Non dosable | 116% |
| | LPS | | | Non dosable | 110% |
| | Staurosporine | | | | 53% |
| | | | | | |
| | | 3 h | 3 h | | |
| | Control | 619.4 | 576.7 | Non dosable | |
| | MU5 | 655.6 | 1097.8 | Non dosable | |
| | G3 | 664.3 | 679.2 | Non dosable | |
| | T2 | 636.4 | 583.1 | Non dosable | |
| | T12 | 698.4 | 677.1 | Non dosable | |
| | The sign * in the Tab. 1 identifies the values that are significantly high with respect to the control. | | | | |

As expected, no production of IL-1 was identified, as already noted by Graham (2004), according to whom the human keratinocytes express very low levels of such pro-inflammatory cytokine. Significant increases of IL-1 were actually noted as a consequence of contact with P. *acnes* (Graham 2004), which generates a considerable pro-inflammatory effect. On the contrary, the contact of lactobacilli with the keratinocytes has not determined any significant variation in the levels of IL-1) (Tab. 1).

Some of the tested bacterial strains have instead determined, as a consequence of contact, a considerable increase in the expression of anti-inflammatory cytokines. In particular, MU5 and T12 have shown a significant anti-inflammatory capacity through the induction of IL-10 and IL-6, especially after a long exposure time. The anti-inflammatory response induced by the treatment with some lactobacilli is potentially interesting from the application point of view, due to the possible counterbalance of the pro-inflammatory action performed by some skin pathogens.

Moreover, a potentially interesting element is represented by the induction, by the tested lactobacilli, of the release of TGF-ß (transforming growth factor), known initiator of the granulated tissue formation within the skin repair process of wounds. The increase of its release could then represent a precious input for the skin re-epithelization. However, the literature reports controversial data concerning the participation of TGF-ß in the solar elastosis, therefore it is believed that the response to the keratinocytes exposure to the lactobacilli with respect to the induction of such factor must and can be examined better *in vivo.*

### 1.2.2 Production of metabolites

Some species of the Lactobacillus spp. kind are capable of producing hydrogen peroxide if exposed to the air. The production of hydrogen peroxide could be therefore expressed by living lactobacilli put in contact with the skin surface and thus exposed to oxygen. The capacity to release H₂O₂ to the environment was evaluated quantitatively *in vitro* of the strains being the subject of the present analysis.

The used determination method has made reference to what was described by Yap et al. (2000). The bacterial cultures were incubated for 1-8 hours at 37°C in order to imitate various contact times of the microorganisms to be tested with the skin surface. Then, the cultures were subjected to a decimal count of the living cells and to the H₂0₂ measuring by means of colorimetric test, as described by Yap et al. (2000).

**TABLE 2**

| **Incubation time (hours)** | H₂0₂ released (Ng/10⁹ CFU5) | | | | | | | | |
|---|---|---|---|---|---|---|---|---|---|
| | *L.johnsonii* | *L. gasseri* | *L.plantarum* | *L.paracasei* | *L.crispatusL.* | *gasseri* | *L.paracasei* | *L.gallinarum* | *L. rhamnosus* |
| | ATCC 33200T | DSM 20243T ATCC | 21028 | NCDO 151T | MU5 | T2 | 6/1 | G3 | *T12* |
| 1 | 22.10 | 15.67 | 0.00 | 0.00 | 47.44 | 14,55 | 0,00 | 12,00 | 0,00 |
| 2 | 24,50 | 17,04 | 0,06 | 0,03 | 35,33 | 16,50 | 0,01 | 13,65 | 0,00 |
| 4 | 30,16 | 22,56 | 0,06 | 0,06 | 8,33 | 19,23 | 0,04 | 17,21 | 0,03 |
| 6 | 35,22 | 25,98 | 0,07 | 0,06 | 9,27 | 21,47 | 21,47 | 20,97 | 0,05 |
| 8 | 37,40 | 28,55 | 0,13 | 0,08 | 7,98 | 25,98 | 25,98 | 24,76 | 0,08 |

The data illustrated in the Tab. 2 show that the strains Mu5, T2 and G3 have presented considerable levels of hydrogen peroxide release in the supernatant. The hydrogen peroxide accumulation by species of the "*acidophilus*" group, such as *L. gallinarum, L. crispatus* and *L. gasseri*, corresponds to the characteristics found also in the literature regarding the species that produce H₂0₂ as a consequence of the aerobic metabolism, but are not capable of detoxicating it, since they lack the cellular structures for H₂0₂ degradation.

On the contrary, other considered species have not produced detectable levels of H₂0₂ even after the aerobic exposition.

### 1.3 In vitro evaluation of the induction of the PPAR mechanism by means of use of cellular lines of keratinocytes

The aim of this step was assessment of the expression level, by a monolayer of human keratinocytes of some biomarkers as a consequence of the contact with the bacterial cultures in stationary phase. The used functional procedures were of two types: a) Human keratinocytes put in culture were incubated with different strains of lactobacilli (M01 50) for 1 hour. Then, the cells were washed, the growth medium restored and the cellular culture prolonged for further 4 hours; b) Human keratinocytes put in culture have been pre-treated with a solution containing lipopolysaccharides (LPS 10ug/m1) for 30 minutes and subsequently incubated with different strains of lactobacilli (M01 50) for 1 hour. Then, the cells were washed, the growth medium restored and the cellular culture prolonged for further 4 hours. In both cases (a) and (b), after the 4 hours, the cells were collected and lysed in RIPA buffer. Twenty
of the total so obtained proteins were fractionated by means of SDS-PAGE (7,5% w/v) and transferred to the PVDF membrane. In order to evaluate the expression of various proteins, such membrane was then incubated with antibodies specific for recognition of PPARy, PPARα, PPARδ and COX-2.

The choice to evaluate the degree of activation of the peroxisome proliferator-activated receptors (PPARs) was connected to the fact that such transcription factors are usually expressed also at the level of the skin, not only in numerous other organs, in human beings and in animals and take part in a plurality of processes of fundamental importance for maintaining skin homeostasis and the correct differentiation of sebaceous glands (Di Poi, 2004).

The cyclooxygenase (COX-2) is a protein, expressed also at the level of the skin, which is considered responsible, in case of over-expression, for induction of inflammatory phenomena. Actually, the COX-2 represents the target for most of the treatments with non-steroidal anti-inflammatory drugs. The over-regulation of the COX-2 and the related increase of the synthesis of prostaglandins is considered a key factor in the skin tumorigenesis (Elmets 2002; Fischer 2004).

### 1.3.1. Determination of the PPAR-y expression

The expression level of PPAR-y in the keratinocytes exposed to the bacteries has not shown significant variations for any of the tested strains with respect to the control, either in the pre-treatment case (B) with LPS, or without it (A).

### 1.3.2. Determination of the PPAR-α expression

The expression level of PPAR-α in the keratinocytes exposed to the bacteria has not shown significant variations for most of the tested strains with respect to the control, either in the case of pre-treatment (B) with LPS, or without it (A). Only the strain T2 has determined an increase in the PPAR-α expression in the keratinocytes exposed to the bacterium without the pre-treatment with LPS.

### 1.3.3. Determination of the PPAR-δ expression

No effect of significant expression increase of the above mentioned marker was revealed.

### 1.3.4 Determination of the CO-X-2 expression

The strains G3 and T12 have drastically reduced the basal levels of this enzyme, while the T2 seems to act only partially. An induction of the COX2 was found as a result of the treatment with LPS. Such bacterial action has a considerable interest in the control of the over-regulation of the COX-2 enzyme associated to the skin inflammatory phenomena.

The tests described at points 1.3.1 to 1.3.4 were repeated for the strain T12 that has provided significant indications during the previous tests, extending the exposure times of the keratinocytes to the bacterial cells respectively to 2 hours and 6 hours, in order to verify whether the missing variation of the levels of PPAR receptors is connected to the reduced induction time used during the tests. The obtained results reveal that, while the PPAR-y and PPAR-δ levels are not significantly modified by the action of the bacterial induction, the levels of PPAR-α undergo a considerable variation with respect to the control, for extended treatment times. In light of what was indicated by Di Poi (2004) about the PPAR-α role in the formation of the skin barrier, the action carried out by the strain T12 confirms to have a considerable interest in the promotion of the epidermic resistance to the action of the environmental agents. The key role of the PPAR-α in the skin homeostasis is demonstrated also by the existence of patents that protect the use of drugs, which, acting as receptors of such activator, are used in the treatment of the skin alterations (US Patent no. 6060515 of 2000).

Also the expression level of ξ-cadherin was evaluated in order to reveal possible variations in the expression of this protein, which performs an important connection function between the epithelial cells. No significant variation was detected.

The variations of the expression levels of the PPARs receptors were further evaluated by means of the fluorescence microscope observation, which has confirmed the induction response of the PPAR-α expression as a result of contact of the keratinocytes with the strain T12 for 1 hour, while the receptors Y and ß have not shown significant variations. The keratinocytes were treated with specific antibodies against the above mentioned receptors with subsequent detection of the fluorescence signal.

### 1.4 In vitro assessment of the inhibitory action of the skin pathogens

The following pathogenic bacteria were considered for the in vitro inhibition tests

*Candida albicans* SKF 2270
*Staphylococcus aureus* ATCC 19636
*Staphylococcus epidermidis* (clinical isolate)
*Streptococcus pyogenes* SKF 13400
*Enterococcus faecalis* (clinical isolate)

The tests were carried out according to what is described by Coconnier et al. (1997) by including the pathogens in plate and checking the inhibitory capacity of the bacterial strains to be tested (both as a washed culture and as a supernatant) with respect to the pathogens growth.

The measure of the inhibiting areola has represented the reference parameter for assessment of the inhibiting activity of the supernatant.

The control was the *Planobispora* rosea mould which has known inhibiting activity. The negative control was obtained with the physiological saline.

The results have revealed a slight inhibiting action for the strains MU5 and G3 with respect to *S. aureus* and for the strains T12 and G3 with respect to *S. pyogenes*, whereas no significant activity was found with respect to the other tested pathogens.

The previously described results were summarized in Table 3, assigning an asterisk following to a positive result obtained for a specific bacterial strain. The sum of the asterisks assigned to each strain was the key of the decision making process.

**Table 3 - Decision making report**

| | Immune activation | | | H₂O₂ | PPARs | | | COX-2 | Pathogens inhibition | Total points |
|---|---|---|---|---|---|---|---|---|---|---|
| | II-6 | IL-10 | TGF-ß | | a | 6 | y | | | |
| Lactobacillus gasseri T2 | X | | | X | | | | | | 2 |
| Lactobacillus paracasei 6/11 | | | | | | | | | | 0 |
| Lactobacillus gallinarum G3 | X | | | X | | | | X | X | 4 |
| *Lactobacillus rhamnosus* T12 | X | X | | | X | | | X | X | 5 |
| Lactobacillus crispatus MU5 | X | X | X | X | | | | | X | 5 |

The strains, T12 and MU5 have revealed the characteristics of analytical interest, with particular reference to the strain T12. The latter has appeared the only strain capable of interacting in a significant way with the PPAR receptors. A possible explanation of the revealed anti-inflammatory activity seems to be connected to this property, partly ascribable to an action mechanism typical only for this strain.

### 1.4 Verification of suitable formulations,

The components that are critical for the bacterial stability and the possible strategies for ensuring the product appropriate shelf-life were identified.

Some substances, listed later on (Step 3) were tested as for the survival of the selected strains. The potential technological difficulties associated to the use of a living lactobacillus for obtaining a stable preparation for topical use, were by-passed testing the efficiency of the selected strain (*L.rhmanosus* T12) in a partially heat-inactivated form.

### 3.1 Providing bacterial lyophilisates for preliminary tests

Two bacterial lyophilisates were prepared as test formulations.

The characteristics of the lyophilisates are listed in the following:
*Lactobacillus rhamnosus* 75 g 7.9 x 109 CFU/g
*Lactobacillus acidophilus* 49 g 3.0 x 1010 CFU/g

Subsequent activities provided for the test formulations, to be prepared during the Step 1 carrying out, were transferred to a potentially "final" formulation, based on the use of the strains T12 or MU5. For this purpose, the activities described in the following paragraphs were carried out.

### Stability tests of the cultures of bacterial strains selected in oily matrices.

The selected bacterial strains were cultivated in selective synthetic ground and the obtained cells were washed twice with physiological saline in order to remove the exhausted culture ground.

The so obtained cultures were re-suspended in physiological water and subjected to decimal count to determine the concentration in living cells.

Then, the cultures were mixed, by vigorous shaking, with oily matrices and the so obtained emulsions were preserved at room temperature in order to create environmental conditions that simulate the commercial product preservation and to put the bacterial cells in the worst hydration conditions.

The stability results obtained after 7, 14 and 30 days of preservation were summarized in the following Table 4.

The CB compound was excluded from the test due to its corrosiveness with respect to the laboratory material used for the tests.

Table 4 - Loss of vitality (in log10 CFU) of the 3 bacterial strains tested after 7 (A), 14 (B) and 30 days (C) of preservation at room temperature in hydration conditions in emulsion with oily matrices

**Time A**

| | *L. rhamnosus* T12 | *L. crispatus* MU5 |
|---|---|---|
| Matrix | loss in log10 CFUs | loss in log10 CFUs |
| OG | 6.49 | 5.56 |
| VB | 6.49 | 5.56 |
| PEH | 6.49 | 5.56 |
| ST | 6.49 | 5.56 |
| CB | nd | nd |
| GE | 4.01 | 5.56 |
| N | 2.01 | 5.56 |
| DC | 4.71 | 5.56 |
| BK | 6.49 | 5.56 |

**Time B**

| | *L. rhamnosus* T12 | *L. crispatus* MU5 |
|---|---|---|
| Matrix | loss in log10 CFUs | loss in log10 CFUs |
| OG | 6.49 | 6.56 |
| VB | 6.49 | 6.56 |
| PEH | 6.49 | 6.56 |
| ST | 6.49 | 6.56 |
| CB | nd | nd |
| GE | 4.17 | 6.56 |
| N | 2.49 | 6.56 |
| DC | 4.89 | 6.56 |
| BK | 6.49 | 6.56 |

**Time C**

| | *L. rhamnosus* T12 | *L. crispatus* MU5 |
|---|---|---|
| Matrix | loss in log10 CFUs | loss in log10 CFUs |
| OG | 6.49 | 6.56 |
| VB | 6.49 | 6.56 |
| PEH | 6.49 | 6.56 |
| ST | 6.49 | 6.56 |
| CB | nd | nd |
| GE | 5.49 | 6.56 |
| N | 6.49 | 6.56 |
| DC | 5.49 | 6.56 |
| BK | 6.49 | 6.56 |

The used matrixes identified with the abbreviations are constituted by the following oily matrices:
OG: wheat germ oil
VB: white vaseline
PEH: ethyl-2-hexyl palmitate
ST: SI TEC DM100 dimethicone
CB:: cetyol β-dibutyl adipate
GE: SF1202-GE cyclomethicone
N: Nesatol C10-18 triglycerides
DC: Dow Corning 9041 (dimethicone + dimethicone cross-linked polymer)
BK: karité butter.

The extreme conditions applied to the preservation of the selected strains (hydration, exposure to the light and high temperature) have determined a sharp reduction in vitality for some of used matrices from the very first days of storage.

The MU5 strain has revealed a strong instability in all the tested substances, in accordance with the characteristics of sensibility to the environmental stress of the species it belongs to. Consequently, this strain appears not suitable for the topical formulations.

On the contrary, the strain T12 has revealed a contained loss of vitality only in the Nesatol C10-18 triglycerides matrix until the 14^{th} day with the subsequent significant drop of living cells.

### Preparation of lyophilisates of the selected bacterial strains on the laboratory scale

Considering the results of the previous stability tests, it was believed advisable to prepare lyophilisates of the bacterial strain T12, while the strain MU5 was excluded, since it has demonstrated scarce resistance in the environmental conditions, in order to evaluate the miscibility of the bacterial powders with the compounds used in the cosmetic formulations and the related stability, expressed as the number of living cells.

The obtained lyophilisates have the following load:
*- L. rhamnosus* T12 130 g 2,0 x 1011 CFU/g

The lyophilized bacterial powders appeared appropriate to be mixed with other typical components for the formulation of topical compounds.

The Italian priority patent application MI 2009A001547 was filed on 8 September 2010 by the same Applicant.

### EXAMPLES

The following examples are provided as a pure illustration of the present invention and should not be intended as limiting of the protection scope, as it appears from the appended claims.

### Example 1

### Detergent composition suitable for external uses

| Component (INCI name) | Quantity w/w (%) |
|---|---|
| Sodium lauryl glutamate | 1.00-6.00 |
| Sodium lauryl sarcosinate | 1.00-6.00 |
| PEG-120 Methyl glucose dioleate | 1.00-4.00 |
| Glycol distearate | 0.20-1.00 |
| Sodium laureth sulfate | .0.20-1.00 |
| Myrystyl alcohol | 0.20-1.00 |
| Cocamydopropyl betaine | 0.50-1.00 |
| Sodium cocoyl glutamate | 1.00-6.00 |
| Disodium EDTA | 0.025-0.20 |
| *Lactobacillus rhamnosus* T12 lysate (obtained from the treatment of a lyophilisate containing 1*10 exp 11 ufc) | 0.001-0.50 |
| Maltodextrin | 0.01-0.50 |
| PEG-60 Maracuja glycerides | 0.50-1.50 |
| Phenoxyethanol | 0.70-0.90 |
| Methylparaben | 0.10-0.20 |
| Propylparaben | 0.01-0.04 |
| Parfum | 0.30-0.40 |
| Aqua | *q.s.* 100.00 |

### Example 2

| Roll-on Deodorant composition | |
|---|---|
| Component (INCI name) | Quantity w/w (%) |
| Steareth-2 | 1.00-3.00 |
| Steareth-21 | 1.00-1.50 |
| Glycerin | 1.00-4.00 |
| PPG-15 Stearyl ether | 1.00-5.00 |
| Aluminium chloridrate | 10.0-17.0 |
| *Lactobacillus rhamnosus* T12 lysate (obtained from the treatment of a lyophilisate containing 1*10 exp 11 ufc) | 0.001-0.50 |
| Maltodextrin | 0.01-0.50 |
| Phenoxyethanol | 0.70-0.90 |
| Methylparaben | 0.10-0.20 |
| Propylparaben | 0.01-0.04 |
| Parfum | 0.50-0.60 |
| Aqua | *q.s.* 100.00 |

### Example 3

| Ointment | |
|---|---|
| Component (INCI name) | Quantity w/w (%) |
| Paraffinum liquidum | 1.00-5.00 |
| PEG-8 | |
| PEG-40 | |
| PEG-75 | 1.00-10.00 |
| Hydrogenated polysobutene | 1.00-10.00 |
| PPG-15 Stearyl ether | 1.00-3.00 |
| *Lactobacillus rhamnosus* T12 lysate (obtained from the treatment of a lyophilisate containing 1*10 exp 11 ufc) | 0.001-0.50 |
| Maltodextrin | |

### Example 4

**LEAVE ON DETERGENT**

| Component (INCI name) | Quantity w/w (%) |
|---|---|
| Propylene glycol | 1.00-4.00 |
| *Lactobacillus rhamnosus* T12 lysate (obtained from the treatment of a lyophilisate containing 1*10 exp 11 ufc) | 0.001-0.50 |
| Maltodextrin | 0.01-0.50 |
| Paraffinum liquidum | 1.00-5.00 |
| PEG-8 Beeswax | 1.00-5.00 |
| Xanthan gum | 0.10-0.40 |
| Ceatearyl alcohol | 1.00-4.00 |
| Oryza sativa cera | 0.10-1.50 |
| Citric acid | 0.10-0.30 |
| Ammonium glycirrhizate | 0.001-0.01 |
| Sodium hydroximethylglycinate | 0-10-0.50 |
| Phenoxyethanol | 0.70-0.90 |
| Aqua | q.s. 100.00 |

### Example 5

**AFTERSHAVE FLUID CREAM**

| Component (INCI name) | Quantity w/w (%) |
|---|---|
| Glycerin | 1.00-4.00 |
| Propanediol | 1.00-3.00 |
| PEG-100 Stearate | 0.10-0.40 |
| Glyceryl stearate | 0.10-0.40 |
| Xanthan gum | 0.10-0.40 |
| Ceatearyl alcohol | 0.10-0.40 |
| Disodium EDTA | 0.10-0.40 |
| *Lactobacillus rhamnosus* T12 lysate (obtained from the treatment of a lyophilisate containing 1*10 exp 11 ufc) | 0.001-0.50 |
| Maltodextrin | |
| Hydrogenated polydecene | 1.00-5.00 |
| Caprilic/capric triglycerides | 1.00-5.00 |
| Butyrospermum parkii | 1.00-5.00 |
| Meadowfoam (Limnanthes alba) seed oil | 1.00-3.00 |
| Dimethicone | 1.00-3.00 |
| Sodium hydroximethylglycinate | 0-10-0.20 |
| Phenoxyethanol | 0.70-0.90 |
| Lactic acid | to pH 5.5 |
| Parfum | 0.30 |
| Delta tocopherol | 0.02-0.25 |
| Sorbityl furfural | 0.10-0.90 |
| Aqua | 100.00 |

### Example 6

**BODY MILK**

| Component (INCI name) | Quantity w/w (%) |
|---|---|
| Glycerin | 1.00-6.00 |
| Propylene glycol | 1.00-6.00 |
| Cetyl hydroxyethylcellulose | 0.10-0.40 |
| Xanthan gum | 0.10-0.40 |
| Tapioca starch | 1.00-2.00 |
| Disodium EDTA | 0.025-0.20 |
| *Lactobacillus rhamnosus* T12 lysate (obtained from the treatment of a lyophilisate containing 1*10 exp 11 ufc) | 0.001-0.50a |
| Maltodextrin | 0.01-0.50 |
| Sorbitan stearate | 2.00-5.00 |
| Sucrose cocoate | 0.10-1.00 |
| Ethylexyl palmitate | 1.00-5.00 |
| Hydrogenated polydecene | 100-5.00 |
| Caprilic/capric triglycerides | 1.00-5.00 |
| Butyrospermum parkii | 1.00-5.00 |
| Meadowfoam (Limnanthes alba) seed oil | 1.00-3.00 |
| Dimethicone | 1.00-3.00 |
| Sodium hydroximethylglycinate | 0-10-0.20 |
| Phenoxyethanol | 0.70-0.90 |
| Lactic acid | q.s. |
| Parfum | 0.30 |
| Delta tocopherol | 0.02-0.25 |
| Sorbityl furfural | 0.10-0.90 |
| Aqua | q.s. 100.00 |

### Example 7

**CREAM especially for face**

| Component (INCI name) | Quantity w/w (%) |
|---|---|
| Glycerin | 2.00-5.00 |
| Methylpropanediol | 0.20-2.00 |
| Acrylates/C10-30 Alkyl acrylate crosspolymer | 1.00-2.00 |
| Ceatearyl alcohol | 0.20-2.50 |
| Cetearyl glucoside | 0.20-2.50 |
| PEG-100 Stearate | 0.20-1.00 |
| Sodium hyaluronate | 0.05-0.5 |
| Disodium EDTA | 0.10-0.50 |
| *Lactobacillus rhamnosus* T12 lysate (obtained from the treatment of a lyophilisate containing 1*10 exp 11 ufc) | 0.001-0.50 |
| Maltodextrin | 0.01-0.50 |
| Cetyl palmitate | 0.50-3.00 |
| Hydrogenated Evening Primrose Oil | 0.50-3.00 |
| Polybutene | 0.50-3.00 |
| Hydrogenated castor oil | 1.00-4.00 |
| Dicaprylyl ether | 1.00-4.00 |
| Butyrospermum parkii | 1.00-5.00 |
| Beta sitosterol | 0.10-0.50 |
| Delta tocopherol | 0.05-0.20 |
| Dimethicone | 0.50-1.50 |
| Dimethicone crosspolymer | 0.10-1.50 |
| Sorbityl furfural | 0.5-1.00 |
| Sodium hydroximethylglycinate | 0-25-0.50 |
| Parfum | q.s. |
| Aqua | 100.00 |

### Example 8

**DIETARY PRODUCT - SOFT GELATIN CAPSULE**

| | |
|---|---|
| Each soft gelatin capsule (pearl) contains: | quantity u.m. |
| *Lactobacillus rhamnosus* T12 | 1*10 exp 9 CFU |
| Insoluble natural fiber | 5-100 mg |
| Vitamine E (dl-alpha tocopherol) | 5-100 mg |
| Soya bean oil | 250 mg |
| Soya bean lecithin | 5 mg |
| Mono- and diglycerides of fatty acids | 30 mg |
| Shell constituents: | |
| Gelatin | 145 mg |
| Glycerol | 67 mg |

### Example 9

**DIETARY PRODUCT - TABLETS**

| | |
|---|---|
| Each tablet contains | quantity u.m. |
| *Lactobacillus rhamnosus* T12 | 1*10 exp 9 CFU |
| Insoluble natural fiber | 5-100 mg |
| Methionine | 200 mg |
| Microcrystalline cellulose | 100-250 mg |
| Anhydrous dibasic calcium phosphate | 100-200 mg |
| Hydroxypropylmethylcellulose | 30-100 mg |
| Chelate amino acid zinc for a quantity of Zn | 15 mg |
| Ascorbic acid | 60 mg |
| Vitamin E acetate | 15 mg |
| Mono- and diglycerides of fatty acids (E471) | 5.0-10.0 mg |
| Silicon dioxide (colloidal silica) | 5.0-10.0 mg |
| Biotin | 0.23 mg |

### Example 10

**DIETARY PRODUCT - HARD GELATIN CAPSULE**

| | |
|---|---|
| Each hard gelatin capsule contains: | quantity u.m. |
| *Lactobacillus rhamnosus* T12 | 1*10 exp 9 CFU |
| Nicotinamide (Vitamin B3) | 18 mg |
| Maltodextrins | 5-50 mg |
| Insoluble natural fiber | 5-100 mg |
| Magnesium stearate | 1-10 mg |
| Silicon dioxide | 3-6 mg |
| Natural gelatin | outer shell |

### Example 11

**DIETARY PRODUCT - OROSOLUBLE GRANULES**

| | |
|---|---|
| Each bag contains: | quantity u.m. |
| Tyndallized of *Lactobacillus rhamnosus* T12 (obtained by the treatment of a lyophilisate containing 1*10 exp 11 CFU) | 100 mg |
| Inulin | 5-100 mg |
| Ascorbic acid | 50-60 mg |
| Fructose | 0.5-3.0 g |
| Maltodextrins | 0.5-3.0 g |
| Malic acid | 1-10 mg |
| Aroma | 10.0-50.0 mg |
| Sucralose | 0.005 mg |

### Example 12

**DIETARY PRODUCT - SINGLE-DOSE BAGS (MIXTURE OF POWDERS) TO DISSOLVE IN WATER**

| | |
|---|---|
| Each bag contains: | quantity u.m. |
| *Lactobacillus rhamnosus* T12 | 1*10 exp 9 CFU |
| Insoluble natural fiber | 5-100 mg |
| Nicotinamide | 10-18 mg |
| Ascorbic acid | 50-60 mg |
| Methionine | 100-200 mg |
| Extract from cellular culture of Ajuga reptans | 2.50 mg |
| Biotin | 0.25 mg |
| Chelate aminoacid zinc for a quantity of Zn | 15 mg |
| Selenium yeast for a quantity of Se | 0.05 mg |
| Fructose | 0-3 g |
| Maltodextrins | 0-3 g |
| Malic acid | 0-100 mg |
| Sucralose | 0.005 mg |
| Aroma | 0.25 mg |

## Claims

1. A *Lactobacillus rhamnosus* strain having accession number LMG P-25211.

2. A *Lactobacillus rhamnosus* strain in accordance with claim 1 for use in the treatment of an inflammatory and/or allergic skin affection or disease.

3. A composition or food supplement for oral administration comprising an effective amount of *Lactobacillus rhamnosus* having accession number LMG P-25211 and a physiologically acceptable carrier.

4. A composition or food supplement in accordance with claim 3 for use in the treatment or prevention of an inflammatory or allergic skin affection or disease.

5. A composition or food supplement in accordance with claim 4 wherein said inflammatory or allergic skin affection is selected from acne, atopic dermatitis, seborrhoeic dermatitis, eczema, sensitive skin.

6. A composition for local use comprising an effective amount of *Lactobacillus rhamnosus* having accession number LMG P-25211 and a physiologically acceptable carrier.

7. A composition for local use in accordance with claim 6 **characterised in that** it is in a form selected from cream, emulsion, ointment, gel, lather, liniment, powder, aqueous solution or suspension, oily solution or suspension or biphasic solution or suspension and mixtures thereof.

8. A composition in accordance with claim 6 or 7 **characterised in that** the composition is a cosmetic composition or a pharmaceutical composition.

9. A composition in accordance with claim 6 for use in a method for the anti-inflammatory treatment through induction of IL-6 and/or IL-7 interleukin.

10. A composition in accordance with claim 6 or 7 for use in the treatment or prevention of an inflammatory and/or allergic skin affection.

11. A composition for use in accordance with claim 10 wherein said inflammatory and/or allergic skin affection is selected from among acne, atopic dermatitis, seborrhoeic dermatitis, eczema, sensitive skin, cutaneous rash, erythema.

12. A composition in accordance with claim 3 or 6 for use in a method for the anti-inflammatory treatment through the expression of the anti-inflammatory PPARy protein.

13. A composition in accordance with claim 3 or 6 for use in a method for the anti-inflammatory treatment through a COX-2 level reduction.

## Patentansprüche

1. *Lactobacillus rhamnosus*-Stamm mit der Hinterlegungs-Nummer LMG P-25211.

2. *Lactobacillus rhamrtosus*-Stamm nach Anspruch 1 zur Verwendung bei der Behandlung eines/einer entzündlichen und/oder allergischen Hautleidens oder -erkrankung.

3. Zusammensetzung oder Nahrungsergänzungsmittel zur oralen Verabreichung, die/das eine wirksame Menge an *Lactobacillus rhamnosus* mit der Hinterlegungs-Nummer LMG P-25211 umfasst.

4. Zusammensetzung oder Nahrungsergänzungsmittel nach Anspruch 3 zur Verwendung bei der Behandlung oder der Vorbeugung eines/einer entzündlichen und/oder allergischen Hautleidens oder -erkrankung.

5. Zusammensetzung oder Nahrungsergänzungsmittel nach Anspruch 4, worin das entzündliche oder allergische Hautleiden ausgewählt ist aus Akne, atopischer Dermatitis, seborrhoischer Dermatitis, Ekzem, empfindlicher Haut.

6. Zusammensetzung zur lokalen Verwendung, die eine wirksame Menge an *Lactobacillus rhamrtosus* mit der Hinterlegungs-Nummer LMG P-25211 und einen physiologisch annehmbaren Träger umfasst.

7. Zusammensetzung zur lokalen Verwendung nach Anspruch 6, **dadurch gekennzeichnet, dass** sie in einer Form vorliegt, die ausgewählt ist aus Creme, Emulsion, Salbe, Gel, Schaum, Einreibemittel, Puder, wässrige Lösung oder Suspension, ölige Lösung oder Suspension oder zweiphasige Lösung oder Suspension und Mischungen daraus.

8. Zusammensetzung nach Anspruch 6 oder 7, **dadurch gekennzeichnet, dass** die Zusammensetzung eine kosmetische Zusammensetzung oder eine pharmazeutische Zusammensetzung ist.

9. Zusammensetzung nach Anspruch 6 zur Verwendung in einem Verfahren zur anti-inflammatorischen Behandlung durch die Induktion von IL-6- und/oder IL-7-Interleukin.

10. Zusammensetzung nach Anspruch 6 oder 7, zur Verwendung bei der Behandlung oder Vorbeugung eines entzündlichen und/oder allergischen Hautleidens.

11. Zusammensetzung zur Verwendung nach Anspruch 10, worin das entzündliche und/oder allergische Hautleiden ausgewählt ist aus Akne, atopischer Dermatitis, seborrhoischer Dermatitis, Ekzem, empfindlicher Haut, Hautausschlag, Erythem.

12. Zusammensetzung nach Anspruch 3 oder 6 zur Verwendung in einem Verfahren zur anti-inflammatorischen Behandlung durch die Expression des anti-inflammatorischen PPARy-Proteins.

13. Zusammensetzung nach Anspruch 3 oder 6 zur Verwendung in einem Verfahren zur anti-inflammatorischen Behandlung durch eine COX-2-Spiegel-Reduktion.

## Revendications

1. Souche de *Lactobacillus rhamnosus* ayant le numéro d'accès LMG P-25211.

2. Souche de *Lactobacillus rhamnosus* selon la revendication 1, destinée à une utilisation dans le traitement d'une affection ou maladie de la peau inflammatoire et/ou allergique.

3. Composition ou complément alimentaire pour une administration orale, comprenant une quantité efficace de *Lactobacillus rhamnosus* ayant le numéro d'accès LMG P-25211, et un support physiologiquement acceptable.

4. Composition ou complément alimentaire selon la revendication 3, destinée à une utilisation dans le traitement d'une affection ou maladie de la peau inflammatoire et/ou allergique.

5. Composition ou complément alimentaire selon la revendication 4, où ladite affection inflammatoire ou allergique de la peau est choisi parmi l'acné, la dermatite atopique, la séborrhée, l'eczéma et la peau sensible.

6. Composition à usage local comprenant une quantité efficace de *Lactobacillus rhamnosus* ayant le numéro d'accès LMG P-25211, et un support physiologiquement acceptable.

7. Composition à usage local selon la revendication 6, **caractérisé en ce qu'**elle est sous une forme choisie parmi une crème, une émulsion, une pommade, un gel, une mousse, un liniment, une poudre, une solution ou suspension aqueuse, une solution ou suspension huileuse ou une solution ou suspension biphasique, et des mélanges de ceux-ci.

8. Composition selon la revendication 6 ou 7, **caractérisée en ce que** la composition est une composition cosmétique ou une composition pharmaceutique.

9. Composition selon la revendication 6, à utiliser dans un procédé de traitement anti-inflammatoire par l'induction d'interleukine IL-6 et/ou IL-7.

10. Composition selon la revendication 6 ou 7, à utiliser dans le traitement ou la prévention d'une affection de la peau inflammatoire et/ou allergique.

11. Composition à utiliser selon la revendication 10, dans laquelle ladite affection inflammatoire et/ou allergique de la peau est choisie parmi l'acné, la dermatite atopique, la séborrhée, l'eczéma, la peau sensible, une éruption cutanée et un érythème.

12. Composition selon la revendication 3 ou 6, à utiliser dans un procédé pour le traitement anti-inflammatoire par l'expression de la protéine PPARy anti-inflammatoire.

13. Composition selon la revendication 3 ou 6, à utiliser dans un procédé pour le traitement anti-inflammatoire par une réduction du niveau de COX-2.
